# EUROPEAN PATENT SPECIFICATION

(11) **EP 1 923 010 B1**
(45) Date of publication and mention of the grant of the patent: **04.01.2012**
(21) Application number: 07022259.1
(22) Date of filing: 16.11.2007
(51) Int. Cl.: A61B 17/34

(54) **Trocar**
Trokar
Trocart

(30) Priority: 16.11.2006 JP 2006310565
(43) Date of publication of application: 21.05.2008
(73) Proprietor: MANI, INC., Tochigi-ken (JP)
(72) Inventor: Murakami, Etsuo, Utsunomiya-shi Tochigi-ken (JP); Saito, Masahiko, Utsunomiya-shi Tochigi-ken (JP); Yamaguchi, Akio, Utsunomiya-shi Tochigi-ken (JP)
(74) Representative: Patentanwälte Westphal, Mussgnug & Partner

(56) References cited:
- EP-A- 0 972 493
- EP-A- 1 875 873
- US-A- 3 659 607
- US-A- 4 654 030
- US-A- 4 922 602
- US-A- 5 057 082
- US-A- 5 817 061
- US-A- 6 017 356

## Description

### BACKGROUND OF THE INVENTION

### Field of the Invention

The present invention relates to a trocar that is inserted to body tissue to form a passage for delivering a surgical instrument or a tube for introducing a pharmaceutical agent to affected areas in performing surgery.

With reference to a first trocar according to the present invention, a cutting blade 2 of a nearly straight line is formed at the tip of incision members A and C. Thus, an incisional wound is also formed in the shape of a nearly straight line when the tissue is incised, which allows the wound to be easily closed. Therefore, the use of the first trocar enables the time required to seal the incisional wound to be shortened and is advantageous in the surgery that is required to make a cannula portion 10 penetrate the tissue while the tissue is incised with the incision member A that is inserted into and passed through the cannula portion 10.

When a second trocar according to the present invention is used in making the tissue be penetrated by the cannula portion 10 of a cannula B, a small force enables the cannula portion 10 to penetrate the tissue. For this reason, it is not necessary to screw the cannula portion 10 into the tissue, so the tissue is not damaged by dissection. Therefore, the use of the second trocar enables the time required to seal the incisional wound to be shortened and is advantageous in the surgery that is required to make the cannula portion 10 penetrate the tissue.

With reference to a third trocar according to the present invention, the cutting blade 2 of a nearly straight line is formed at the tip of incision members A and C and when making the tissue be penetrated by the cannula portion 10 of a cannula B, a small force enables the cannula portion 10 to penetrate the tissue. Thus, an incisional wound is also formed in the shape of a nearly straight line when the tissue is incised, which allows the wound to be easily closed, and enables the time required to seal the incisional wound to be shortened. In addition, it is not necessary to screw the cannula portion 10 into the tissue, so the tissue is not damaged by dissection. Consequently, the use of the third trocar enables the time required to seal the incisional wound to be shortened and is advantageous in the surgery that is required to make the cannula portion 10 penetrate the tissue.

### Description of Related Art

A trocar has a cylindrical cannula portion and is used to form a passage for passing a surgical instrument and a lighting instrument which are necessary to perform surgery on the affected area, or a tube for introducing a pharmaceutical agent by the steps of incising a part of the tissue on the body surface, making this cannula portion penetrate into the incised site and disposing.

A plurality of trocars, for example, a trocar for inserting a tube that injects physiological saline to maintain intraocular pressure, a trocar for inserting surgical instruments such as forceps and scissors that incise and remove affected parts, and a trocar for inserting a probe for irradiating affected areas with laser beams are used for vitreous surgery in ophthalmology. Such trocars are penetrated a hole created by cutting the conjunctiva and sclera and the tip of cannula portion is inserted into a vitreous body. In this state, necessary instruments or tubes are inserted into the cannula portion of each of the trocars and smooth surgery is performed.

In the vitreous surgery, it is essential to incise the conjunctiva and sclera. Since the cannula portion of the trocar has an extremely small thickness, an incision instrument is inserted into the inside of the cannula portion in advance and the conjunctiva and sclera are incised with the incision instrument and the cannula portion is successively inserted into the vitreous body, and then the incision instrument is pulled out and the cannula portion is left in place.

As an incision instrument to be inserted into the cannula portion of the trocar, for example, an incision instrument produced by grinding the tippart of a roundbar to form threebevels, displacing the tip in which these bevels cross from the center of the round bar and forming a cutting blade with a downward triangular shape (when viewed from the front) by edges, or an incision instrument produced by obliquely grinding the tip of a cylindrical member like an injection needle to form a plurality of bevels, locating the tip in which these bevels intersect with an outer peripheral surface of the cylinder at the outer periphery, and forming a semicircular cutting blade at the outer peripheral surface that intersects with the bevels has been provided.

Particularly, in the case of ophthalmic surgery, it is preferable that incision sites are closed by non-suture technique in order to shorten the recovery period. Thus, the invention described in Japanese Patent Application National Publication (Laid-Open) No. 2003-526461 is proposed. The technique can leave the sclera and/or conjunctiva in a condition that the tissues may seal the openings (incisional wounds) made therein after the surgery in order to perform the intra-ocular surgical procedure. In the technique, the use of a surgical instrument having an operable end with a very small diameter (e.g. 20 to 27 gauge) allows the openings to be small in size, thereby enabling the tissues of openings to be sealed.

In each of the above-mentioned trocars, the tip of the cannula portion to which an incision instrument is inserted is formed in a state that it is cut at a right angle to a shaft line. The tip of the cannula portion is screwed into the conjunctiva and sclera incised with an incision instrument, thereby making the cannula portion penetrate into them.

In the case where the conjunctiva and sclera are incised with the incision instrument in which a cutting blade with a upward or downward triangular shape from the front view is formed or the incision instrument in which a semicircular cutting blade is formed, a section whose incision length to the conjunctiva and sclera is long and whose shape is a triangular or semicircular valve is formed. The section moves like a valve, so it moves easily to the opposing body tissue. As a result, it takes a long time to be closed.

In the invention described in Japanese Patent Application National Publication (Laid-Open) No. 2003-526461, openings formed in the conjunctiva and sclera can be easily sealed because of their small size. However, an inserted portion serving as an incision instrument forms a shoe-horn type of member (paragraph 0020) and the end of the stylet is beveled (e.g., tri-beveled) and sharpened like a needle (paragraph 0079). Therefore, a valve-shaped section having the shape of semicircle or triangle is formed as with a conventional incision instrument even when the technique is used, which remains the problem of requiring time for closure of the tissue.

It is often the case that each of the above-mentioned trocars that are made in the shape in which the tip of the cannula portion is cut at a right angle to a shaft line are used. Thus, there is a possibility of dissecting the tissue when the cannula portion is screwed so as to penetrate to body tissues such as conjunctiva and sclera. The problem is that especially, when the tissue is dissected, it takes a long time to be completely cured.

For that reason, an incision instrument in which the tip of the cannula portion is cut at an angle to a shaft line is also utilized. However, the resistance when making the incisional wound penetrate is still strong and it is used in combination with the conventional incision instrument as described above, therefore the problem of requiring time for closure of the tissue has not been solved. That is, the incisional wound has a valve shape (state in which a valve-shaped section is formed) (because the cannula portion can not be inserted in the case of a straight line wound) since the conventional incision instrument is formed taking into consideration an easy insertion of the cannula portion into the incisional wound, which causes difficulty in closing the wound.

Further, in some cases, the body tissue is first cut using a knife to form an incisional wound which is easily closed and then the incisional wound cut with the knife is enlarged and extended with the incision member of a trocar to insert the cannula portion instead of forming the incisional wound with the trocar alone. However, both knife and trocar are used in the surgery, which causes a problem that the operation becomes complicated.
EP 0 972 493 discloses a trocar comprising an incision member that is detachably inserted into the cannula portion and includes a cutting blade formed at the tip.
US 3 659 607 discloses a trocar comprising a cannula portion and an incision member that is detachably inserted into the cannula portion and includes a cutting blade formed at the tip, which is adapted to perform ophthalmic surgery and wherein a shape of a projection of the tip of the cutting blade onto a plane perpendicular to a longitudinal axis is a straight line or a curved line and the tip part of the cannula portion is formed in a tapered shape.

There is a need for a trocar in which the time required to seal an opening can be shortened, regardless of the size of the opening, by using the trocar alone.

### SUMMARY OF THE INVENTION

According to the present invention, there is provided a first trocar used in performing surgery having a cannula portion into which an incision member including a cutting blade with a shape of a nearly straight line formed at the tip is inserted.

According to the present invention, there is provided a second trocar in which the tip part of a cannula portion into which an incision member is detachably inserted has a slanting cut end and the slanting cut end is formed in a tapered shape.

Further, according to the present invention, there is provided a third trocar that includes a cannula portion in which the tip part of the cannula portion has a slanting cut end and the slanting cut end is formed in a tapered shape and an incision member that is detachably inserted into the cannula portion and includes a cutting blade with a shape of a nearly straight line formed at the tip.

According to the first trocar, the incision member detachably inserted into the cannula portion has the cutting blade with the shape of a nearly straight line formed at the tip, and thus an incisional wound of a nearly straight line is created when incising body tissue by the incision member. For this reason, the section does not open in the shape of a valve. As a result, the state in which the opposing sections are mutually contacted can be maintained and the incisional wound can be easily closed.

According to the second trocar, since the tip part of a cannula portion has a slanting cut end and the slanting cut end is formed in a tapered shape, a sharp part of the tip of the cannula portion passes first and then a thick part passes in sequence when making the cannula portion penetrate the incisional wound formed in tissue with an incision instrument. As a result, the cannula portion can be easily penetrated without screwing it. Therefore, there is no possibility of dissecting the tissue when the cannula portion passes through the opening incised by the incision member.

Since the third trocar includes a cannula portion in which the tip part of the cannula portion has a slanting cut end and the slanting cut end is formed in a tapered shape and an incision member having a cutting blade with a shape of a nearly straight line formed at the tip, it is not necessary to form an incisional wound into which the cannula portion can be easily inserted as a conventional way, namely, a valve-like incisional wound. Accordingly, an incisional wound having a good self-sealing property can be formed and the cannula portion can be easily penetrated to the incisional wound without dissecting the tissue.

Therefore, when the trocar is removed after the surgery, the incisional wound formed in the body tissue is only straight-line cut produced by the incision member. Accordingly, the state in which the opposing sections are mutually contacted can be maintained and incisional wounds can be easily closed.

### BRIEF DESCRIPTION OF THE DRAWINGS

Fig. 1 is a perspective view describing the structure of a trocar;

Fig. 2A is a front view of the trocar;

Fig. 2B is a rear view of the trocar;

Fig. 2C is a right side view of the trocar;

Fig. 3 is a front view in a state that an incision member is inserted into and passed through a cannula as well as a view describing a cutting blade formed at an incision member;

Fig. 4A is a front view of the cannula;

Fig. 4B is a rear view of the cannula;

Fig. 4C is a right side view of the cannula;

Fig. 5A is a front view of the trocar according to a second embodiment;

Fig. 5B is a rear view of the trocar according to the second embodiment;

Fig. 5C is a right side view of the trocar according to the second embodiment;

Fig. 6 is a front view of the trocar according to the second embodiment as well as a view describing a cutting blade formed at an incision member.

### DETAILED DESCRIPTION OF THE PREFERRED EMBODIMENTS

Hereinafter, the most preferred exemplary embodiment of the trocar according to the present invention will be described. According to the present invention, the first trocar includes the incision member for incising body tissue that is detachably inserted into the cannula portion. The incisional wound having the shape of a nearly straight line can be formed in the tissue by forming a cutting blade with a shape of a nearly straight line at the incision member. Thus, the opposing incisional wounds can be mutually approached and easily closed when the cannula portion is removed postoperatively.

In the second trocar, the tip part of a cannula portion has a slanting cut end and the slanting cut end is formed in a tapered shape, which allows for sequentially inserting the cannula portion starting from the slanting cut end without screwing the trocar into the incisional wound in tissue which is formed in a nearly straight line shape with the incision member. The second trocar permits the cannula portion to easily penetrate the tissue by a small force without dissecting the incisional wound with the shape of a nearly straight line by the cannula portion.

Further, the third trocar includes a cannula portion in which the tip part of the cannula portion has a slanting cut end and the slanting cut end is formed in a tapered shape and an incision member having a cutting blade with a shape of a nearly straight line formed at the tip, allowing for forming an incisional wound having the shape of a nearly straight line and a good self-sealing property and making the cannula portion easily penetrate the incisional wound. Furthermore, the incisional wound having a good self-sealing property can be formed by using the trocar alone.

As for the incisionmember of the present invention, the cutting blade of a nearly straight line is formed at the tip in order to incise the tissue having the shape of a nearly straight line. Therefore, it is preferable that the incision member is formed in the shape of a solid rod. For example, in the case where a material of the incision member is a hollow member such as an injection needle, it is difficult to form an incisional wound having a shape of a nearly straight line in the tissue since it may be impossible to form a cutting blade at a hollow portion.

Further, it is preferable that the cutting blade of a nearly straight line to be formed at the tip of the incision member is configured in a nearly straight line passing through the center of rod-like incision member. However, it is not necessarily required that the shape is a nearly straight line passing through the center, and it may be a nearly straight line passing through a position that is off the center or may be a gentle curved line passing through the position that is off the center. Further, the shape may have a structure where two straight lines cross at a gentle angle or a structure that is comprised of a straight and a gentle curved line.

That is, the term "cutting blade of a nearly straight line" and the term "an incisional wound having the shape of a nearly straight line" used herein includes a straight line passing through the center and a straight line passing through a position displaced from the center, or a gentle curved line passing through the center and a gentle curved line passing through a position displaced from the center, or a combination of straight lines or a combination of a straight line and a gentle curved line. Particularly, the length of areas incised by the cutting blade is preferably less than 1.5 times the internal diameter of the cannula. This is because it will get closer to a valve-shaped incisional wound in which a valve-shaped section is formed when the length is longer than the range.

That is, the incision member of the present invention includes a cutting blade not forming a section that can move freely as much as possible, from the viewpoint that it is not preferable to form the valve-shaped section to seal incisional wounds in a short period. The cutting blade may have a curved line shape as long as it does not form the valve-shaped section.

The material for forming the incision member is not particularly limited, and piano wires, steel wires, stainless steel wires, and the like can be selectively used. However, it is preferable to use stainless steel wires with excellent rust prevention properties when the formation of rust in the distribution process is taken into consideration. Particularly, it is preferable to use a wire that is made of austenite stainless steel with high reliability of biocompatibility. Further, it may provide a wire with appropriate elasticity and flexibility as well as a high toughness, to process an austenite stainless steel wire with a predetermined diameter by cold drawing with a predetermined reduction of area to extend the tissue in the shape of fiber.
Consequently, it is preferable to be used as the material of the incision member.

Furthermore, when the cannula portion of the present invention is penetrated to tissue and is placed, it is necessary for the cannula portion to have strength capable of maintaining the shape against the force received from the tissue. In this case, it is possible to use both cannula portions, where one has a strength capable of maintaining the shape when the incision member is pulled out and another one has a deformable degree of strength for that. Examples of the material that can exhibit such strength include metals typified by stainless steel and synthetic resins. Pipes made of such materials can be preferably used. Here, when the instrument inserted into the cannula is moved to use, it is preferable to use the deformable cannula portion.

The angle of a cut end at the tip of the cannula portion to a shaft center of the cannula portion is not limited and any angle may be used so long as the cannula portion can be easily passed through the incisional wound by applying force almost linearly in the direction of the shaft center to the trocar. In the present invention, the cut end is formed in a tapered shape and thus the portion which first contacts the incisional wound of the tissue incised by the incision member is almost point-like and the cannula portion can be easily passed. Therefore, it is possible to make the cannula portion pass through by a small force even when the angle of the cut end is not so acute-angled.

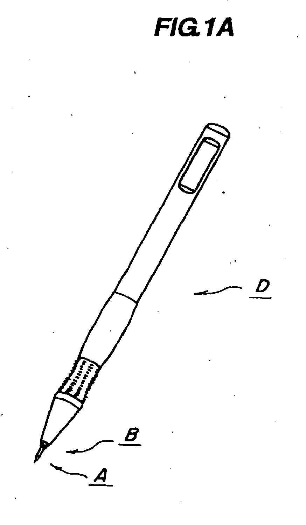

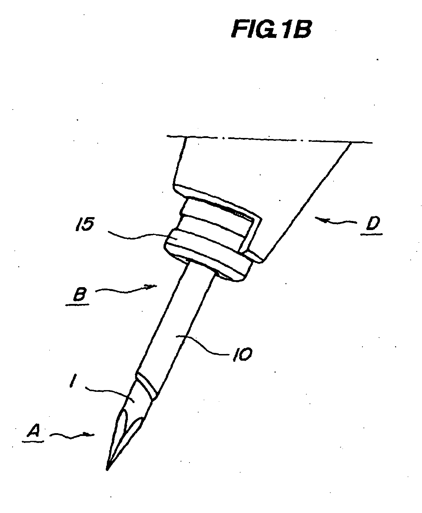

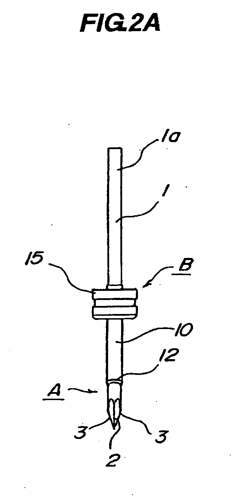

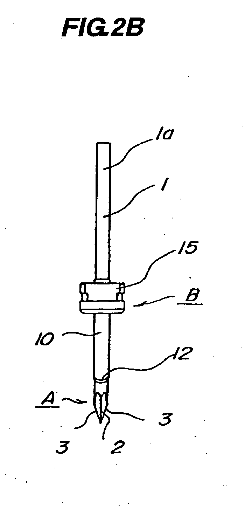

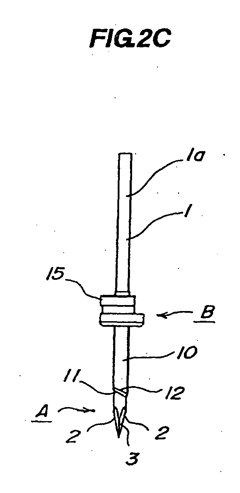

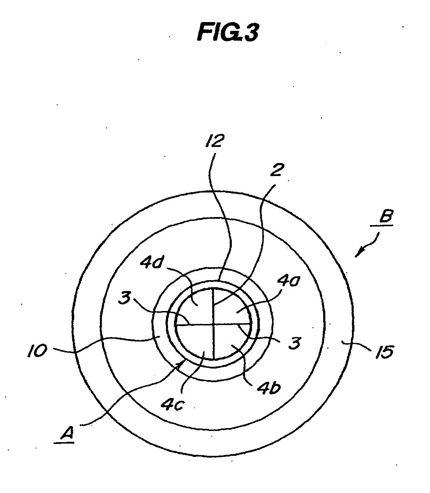

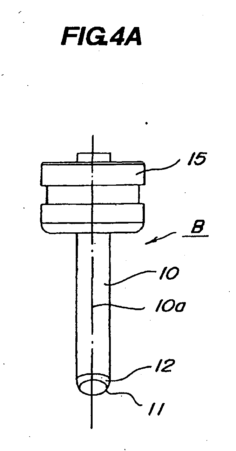

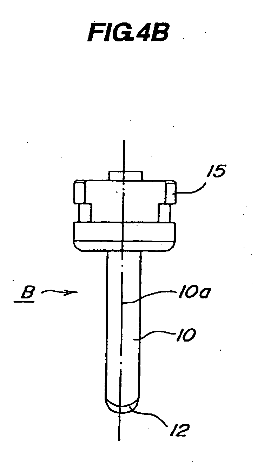

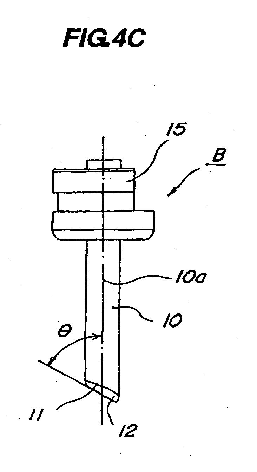

Herein below, a first embodiment of the trocar according to the present invention will be described with reference to the drawings. Fig. 1 is a perspective view describing the structure of the trocar. Fig. 2A is a front view of the trocar. Fig. 2B is a rear view of the trocar. Fig. 2C is a right side view of the trocar. Fig. 3 is a front view in a state that the incision member is inserted into and passed through the cannula as well as a view describing the cutting blade formed at the incision member. Fig. 4A is a front view of the cannula. Fig. 4B is a rear view of the cannula. Fig. 4C is a right side view of the cannula.

The trocar according to the embodiment is directed to use in ophthalmic surgery. That is, it incises the conjunctiva and sclera and then makes a cannula pass through the incisional wound and leave it in place. Thus, surgical instruments or tubes can be inserted by using the cannula.

As shown in Fig. 1, the trocar is detachably equipped with the cannula B in a handle D. The incision member A is detachably inserted into the cannula B. A medical practitioner grasps and operates the handle D and incises the targeted site in living body to form an incisional wound. After inserting the cannula B into the incisional wound, a pore penetrating the incisional wound is formed by detaching the handle D from the cannula B and detaching the incision member A from the cannula B. Then, a surgical instrument or a tube for introducing a pharmaceutical agent can be passed through by using the pore.

Therefore, as shown in Fig. 1A, the handle D has a sufficient length and thickness for the medical practitioner to grasp and operate it. In addition, it is formed in a shape that can be easily grasped. Further, as shown in Fig. 1B, the handle D is attached to the cannula B by engaging the tip of the handle D in a notch and groove which are provided in a socket 15 and the handle D can be detached by releasing the engaging state.

The trocar of the embodiment is configured to treat affected areas by forming an incisional wound in the conjunctiva and sclera in ophthalmic surgery and the incision member A is formed extremely thinly. As a matter of convenience, the embodiment will be described without reference to the handle D, however the medical practitioner grasps the handle D when performing the operation to form an incisional wound using the incision member A.

As shown in Fig. 2, the trocar is configured that the incision member A is detachably inserted into and passed through the cannula B shown in Fig. 4. The incision member A is composed of a solid member having a round-bar shape, and further includes a main body 1 that is formed sufficiently longer than the length of the cannula B and the cutting blade 2 of a nearly straight line that is formed at the tip of the main body 1. The cannula B has rigidity and does not deform easily.

An external diameter of the main body 1 is slightly smaller than an internal diameter of the cannula portion 10 of the cannula B. The main body 1 is formed in the shape of a nearly straight needle. That is, the tip where the cutting blade 2 is formed is configured so that the incision member A can be easily removed from the cannula portion 10 by the medical practitioner's grasp on a tail portion 1a of the opposite side and pulling out it.

However, when the dimensional difference between the outside diameter of the main body 1 and the inside diameter of the cannula portion 10 is too large, the cannula portion 10 is easily detached, which is not preferable. On the other hand, when the dimensional difference is too small, it is difficult to insert the incision member A into the cannula portion 10 or to pull it out. Therefore, it is preferable that the outside diameter of the main body 1 is slightly smaller than the inside diameter of the cannula portion 10. The side of the tail portion 1a that is not inserted into the inner side of the cannula B may be formed in a shape of a straight line from the tip, or in a different shape such as a taper shape. Here, it should be noted that the outside diameter is not limited. When the cannula B has elasticity and a deformable structure, it is also possible to make the outer diameter of the main body 1 larger than the inside diameter of the cannula portion 10.

As shown in Fig. 3, four bevels 4a to 4d are formed at the tip of the main body 1. An edge where the bevels 4a and 4d cross and an edge where the bevels 4b and 4c cross are formed in a nearly straight line, i.e. a straight line passing through the center of the main body 1 and the cutting blade 2 is formed by the edges. Therefore, the cutting blade 2 is formed so as to correspond to a straight line on a diameter passing through the center of the main body 1.

The method for forming four bevels 4a to 4d at the tip of the main body 1 is not particularly limited and it is possible to form each of the bevels 4a to 4d by directly grinding a round bar-like tip of the main body 1. Alternatively, after pressing the round bar-like tip once and forming four base surfaces of bevels 4a to 4d, each of the bevels may be ground.

As mentioned above, it is not necessarily required that the cutting blade 2 is formed so as to correspond to a straight, line passing through the center of the main body 1 and the cutting blade may be a straight line passing through a position displaced from the center, or a gentle curved line passing through a position displaced from the center. Additionally, it may be a combination of two straight lines or a combination of a straight line and a gentle curved line.

Further, an edge where the bevels 4a and 4b cross and edge where the bevels 4c and 4d cross do not have a function to incise tissue and each of the edges is formed as a crest 3 that has just a function to extend the incisional wound formed by the cutting blade 2.

When the cutting blade 2 of a nearly straight line is formed, four bevels 4a to 4d may be formed as that of the embodiment. Alternatively, the cutting blade 2 may be formed with two bevels or three bevels. However, it is possible to actively extend the incisional wound by respectively forming the crests 3 in the direction crossing the cutting blade 2 in the same manner as described in the embodiment. When the cannula portion 10 of the cannula B is inserted into the tissue, it can be easily penetrated by a small resistance, which is advantageous.

As shown in Fig. 4, the cannula B has the cannula portion 10 formed in the cylindrical shape and a socket portion 15 in which the cannula portion 10 is mounted. The cannula portion 10 is composed of a pipe made of metal or a pipe made of synthetic resin with rigidity, and fixed to the socket 15 in a state where a slanting cut end 11 is formed at the tip part and the end part of the other side is fitted in the socket 15.

In surgery of affected areas, the cannula portion 10 passes through and penetrates the incisional wound of tissue which is formed with the incision instrument A and the state is maintained, and the passage for passing a surgical instrument or a tube is formed. Therefore, the cannula portion 10 is formed of a cylindrical member having an appropriate rigidity.

Particularly, a slanting cut end 11 is formed at the tip of the cannula portion 10 and a taper portion 12 is formed at the cut end 11. In the present invention, the angle of the cut end 11 at the tip of the cannula portion 10 to a shaft center 10 of the cannula portion is not limited. The cut end 11 is formed at an angle of about 60 degree in the embodiment.

The taper portion 12 is formed at the external diameter side. The cone angle of the taper portion 12 is not particularly limited and it may be formed at an equal angle to the central shaft of the cut end 11. Thus, the tip of the cannula portion 10 is formed in the point-like shape in which the slanting cut end 11 is combined with the slanting taper portion 12 by forming the slanting cut end 11 at the tip part of the cannula portion 10 as well as forming the taper portion 12 at the cut end 11.

Therefore, when the cannula portion 10 penetrates the incisional wound formed with the incision member A, it is inserted from the extreme tip having the point-like shape. Accordingly, it can be penetrated easily by a small resistance. It is not necessary to rotate and screw the cannula portion 10 in making it penetrate into the incisional wound, resulting in no possibility of dissecting the tissue. Consequently, the healing period of postoperative incisional wound does not require a longer time.

The handle D is detachably equipped with the socket 15. As mentioned above, the cannula B through which the incision member A is passed is attached to the handle D via the socket 15 and then the medical practitioner operates the handle D to form the incisional wound in the tissue with the incision member A. As the incision member A is inserted into the tissue further, the cannula portion 10 is made to penetrate the tissue. Then, the socket 15 is detached from the handle D and further the incision member A is pulled out from the cannula B, thereby leaving the cannula 10 in the incisional wound. A passage to affected areas is formed, allowing for the surgery of affected areas by a surgical instrument and supply of a pharmaceutical agent through a tube by passing through the passage.

Next, the results of the preoperative and postoperative intraocular pressure measured at the time of ophthalmic surgery to form incisional wounds in the conjunctiva and sclera using the trocar in the embodiment will be described and further comparison of preoperative intraocular pressure with postoperative intraocular pressure at the time of forming the incisional wounds using a conventional trocar will be described.

In the case of ophthalmic surgery, there is a need to seal incisional wounds formed in the conjunctiva and sclera without suturing as well as in a short time. It is considered to be preferable that ocular hypotension (7 mmHg or less) should not occur postoperatively and the difference between the intraocular pressure measured before the surgery and the intraocular pressure measured on the day following the surgery is small. That is, the small difference of the intraocular pressure suggests that the closed state of the incisional wounds is good. On the other hand, the large difference of the intraocular pressure suggests that the closed state of the incisional wounds is not good.

The surgery for 29 patients was performed using the trocar in the present embodiment. The mean preoperative intraocular pressure of the 29 patients is 13.8 mmHg. The maximum value was 18.0 mmHg and the minimum value was 10.0 mmHg. When the mean postoperative intraocular pressure of the 29 patients was measured on the day following the surgery, it was 13.2 mmHg. The maximum value was 20.0 mmHg and the minimum value was 8.0 mmHg. From these results, the difference between the mean preoperative intraocular pressure and the mean postoperative intraocular pressure was 0.6 mmHg.

Alternatively, the surgery for 47 patients was performed using a conventional trocar. The mean preoperative intraocular pressure of the 47 patients is 12.9 mmHg. The maximum value was 16.6 mmHg and the minimum value was 9.2 mmHg. When the mean postoperative intraocular pressure of the 47 patients was measured on the day following the surgery, it was 10.0 mmHg. The maximum value was 15.6 mmHg and the minimum value was 4.4 mmHg. From these results, the difference between the mean preoperative intraocular pressure and the mean postoperative intraocular pressure was 2.9 mmHg.

As described above, it was revealed that the intraocular pressure measured on the day following the surgery was decreased to 2.9 mmHg when the conventional trocar was used and the intraocular pressure measured on the day following the surgery remained decreased at 0.6 mmHg when the trocar in the present embodiment was used. Further, ocular hypotension was observed in several patients in the case of the conventional trocar while no ocular hypotension was observed in the patients in the case of the trocar in the embodiment. The results indicate that the trocar in the embodiment is sufficiently advantageous compared with the conventional trocar.

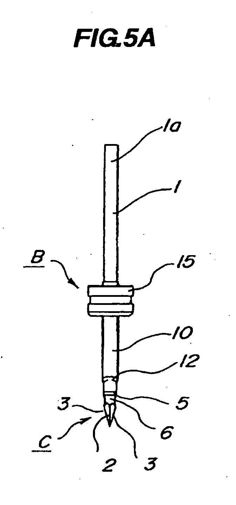

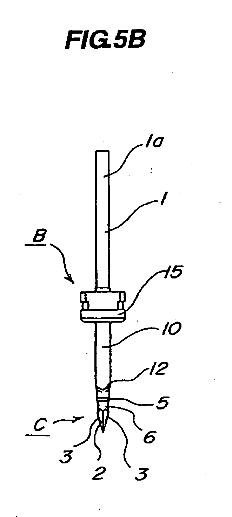

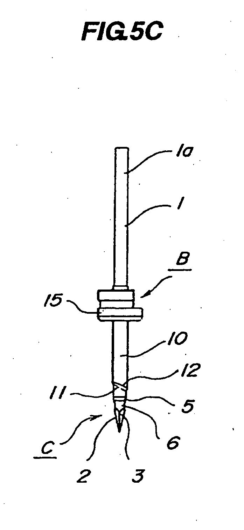

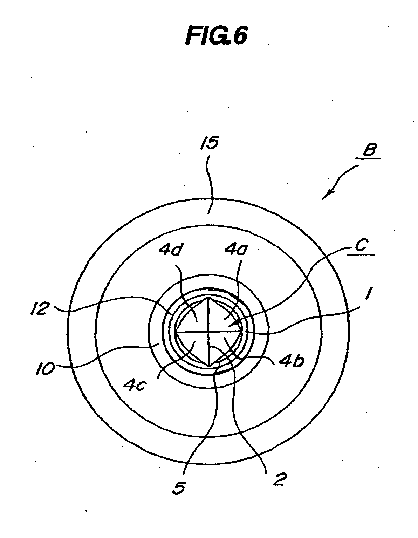

Next, the structure of the trocar according to the second embodiment will be described with reference to the drawings. Fig. 5A is a front view of the trocar according to the second embodiment. Fig. 5B is a rear view of the trocar according to the second embodiment. Fig. 5C is a right side view of the trocar according to the second embodiment. Fig. 6 is a front view of the trocar as well as a view describing a cutting blade formed in an incision member. In this regard, the same numeral references are applied to the same parts and parts with the same functions as in the first embodiment and description will not be repeated here.

In the trocar of the present embodiment, the cannula B has the same structure as the first embodiment. Therefore, the structure of an incision member C will be described.

As shown in Fig. 5, in the incision member C, a taper part 5 is formed at a predetermined position of the tip of the main body 1. A thin shaft portion 6 is formed at the tip of the taper part 5 and the cutting blade 2 is formed at the tip of the thin shaft portion 6. The taper portion 5 is formed at the external diameter side of the main body 1. The thin shaft portion 6 is thinner than the main body 1 due to the provided taper portion 5. Here, the thickness of the thin shaft portion 6 formed through the taper part 5 is not limited and it may be a thickness capable of exhibiting the strength that can sufficiently oppose to the resistance generated when the tissue is cut with the cutting blade 2.

The tip of the main body 1 including the incision member C is pressed once and then four base surfaces of bevels 4a to 4d are formed. Thereafter, respective bevels 4a to 4d are ground to form the cutting blade 2 as well as the crest 3. Additionally, the tip of the main body 1 is formed by particularly pressing the tip part once, which allows for making a portion corresponding to the both ends of the cutting blade 2 and a portion corresponding to the edge of the crest 3 larger than the diameter of the thin shaft portion 6 and nearly equal to the diameter of the main body 1.

Thus, the cutting blade 2 is formed so that the both ends are protruded from the thin shaft portion 6. Therefore, the incision performance can be further improved and the tissue can be cut by a small force.

### CROSS-REFERENCE TO RELATED APPLICATION

This application claims the benefit of priority from the prior Japanese Patent Application No. 2006-310565 filed on Nov. 16th, 2006.

## Claims

1. A trocar, comprising:
a cannula portion (10) having a slanting cut end (11) at a tip part of the cannula portion; and
an incision member (A) that is detachably inserted into the cannula portion, and includes a cutting edge portion (2) having a cutting edge, formed at the tip, said incision member comprising
(a) a main body;
(b) a tip;
(c) a taper portion (5) formed at a predetermined position of the tip side of the incision member; and
(d) a thin shaft portion (6) formed between the tip of the incision member and the taper portion,
wherein the trocar is adapted to be used in ophthalmic surgery,
a shape of a projection of the cutting edge of the incision member onto a plane perpendicular to a longitudinal axis is a straight line or a curved line and
**characterized in that**
the slanting end (11) is formed in a tapered shape, and
a largest diameter of diameters of the tip of the incision member is larger than the diameter of the thin shaft portion and equal to the diameter of the main body of the incision member.

2. The trocar of claim 1, wherein:
(a) the tip of the incision member comprises+ a first to fourth bevels (4a to 4d) that are formed at the tip;
(b) the cutting edge, functioning to incise tissue, being composed of:
(i) a first edge where the first bevel 4a and the fourth bevel 4d cross and
(ii) a second edge where the second bevel 4b and the third bevel 4c cross; and
(c) a crest, that is composed of:
(i) a third edge where the first bevel 4a and the second bevel 4b cross; and
(ii) a fourth edge where the third bevel 4c and the fourth bevel 4d cross;
and wherein the crest does not have a function to incise tissue and has just a function to extend the incisional wound formed by the cutting edge portion
wherein the both ends of the cutting edge and the both ends of the third and fourth edges are larger than the diameter of the thin shaft portion and equal to the diameter of the main body of the incision member.

3. The trocar of claim 1, wherein the angle of the slanting end of the cannula portion is about 60 degree with respect to the shaft center.

4. The trocar of claim 1, wherein the projection of the cutting edge onto the plane perpendicular to the longitudinal axis is any of a straight line passing through the center of the incision member, a straight line passing through a position displaced from the center, and a curved line passing through a position displaced from the center.

5. The trocar of claim 1, wherein the length of the projection of the cutting edge is 1.5 times or less the internal diameter of the cannula.

## Patentansprüche

1. Trokar, umfassend
einen Kanülenabschnitt (10) mit einem schief geschnittenen Ende (11) an einem Spitzenbereich des Kanülenabschnitts; und
ein Einschneideglied (A), das lösbar in den Kanülenabschnitt eingesetzt ist und einen Schneidkantenabschnitt mit einer an der Spitze ausgebildeten Schneidkante aufweist, wobei das besagte Einschneideglied umfasst:
(a) einen Hauptkörper
(b) eine Spitze
(c) einen an einer vorbestimmten Stelle der Spitzenseite des Einschneideglieds ausgebildeten schräg zulaufenden Abschnitt (5); und
(d) einen dünnen Schaftabschnitt (6), der zwischen der Spitze des Einschneideglieds und dem schräg zulaufenden Abschnitt ,
wobei der Trokar für die Verwendung in der Augenchirurgie angepasst ist,
eine Form der Projektion der Schneidkante des Einschneideglieds auf eine zu einer longitudinalen Achse senkrechte Ebene eine gerade Linie oder eine gekrümmte Linie ist und **dadurch gekennzeichnet, dass** das schief geschnittene Ende (11) in einer schräg zulaufenden Form ausgebildet ist und dass ein größter Durchmesser der Durchmesser der Spitze des Einschneideglieds größer als der Durchmesser des dünnen Schaftabschnitts und gleich dem Durchmesser des Hauptkörpers des Einschneideglieds ist.

2. Trokar nach Anspruch 1, wobei
(a) die Spitze des Einschneideglieds erste bis vierte Fasen (4a bis 4d), die an der Spitze ausgebildet sind, aufweist,
(b) die zum Einschneiden von Gewebe dienende Schneidkante gebildet wird durch
(i) eine erste Kante, an der sich die erste Fase 4a und die vierte Fase 4d kreuzen und
(ii) eine zweite Kante, an der sich die zweite Fase 4b und die dritte Fase (4c) kreuzen; und
(c) einen Kamm, der gebildet wird durch
(i) eine dritte Kante, an der sich die erste Fase 4a und die zweite Fase 4b kreuzen und
(ii) eine vierte Kante, an der sich die dritte Fase 4c und die vierte Fase (4d) kreuzen;
und wobei der Kamm nicht die Funktion hat, Gewebe einzuschneiden und lediglich die Funktion hat, die durch den Schneidkantenabschnitt gebildete Schnittwunde zu erweitern;
wobei die beiden Enden der Schneidkante und die beiden Enden der dritten und vierten Kante größer sind als der Durchmesser des dünnen Schaftabschnitts und gleich dem Durchmesser des Hauptkörpers des Einschneideglieds sind.

3. Trokar nach Anspruch 1, wobei der Winkel des schrägen Endes des Kanülenabschnitts ungefähr 60° bezogen auf die Mitte des Schaftes ist.

4. Trokar nach Anspruch 1, wobei die Projektion der Schneidkante auf die Ebene senkrecht zur longitudinalen Achse eine gerade Linie, die durch die Mitte des Einschneideglieds verläuft, eine gerade Linie, die durch eine von der Mitte verschobene Position verläuft oder eine gekrümmte Linie, die durch eine von der Mitte verschobene Position verläuft ist.

5. Trokar nach Anspruch 1, wobei die Länge der Projektion der Schneidkante kleiner oder gleich dem 1.5-fachen des inneren Durchmessers der Kanüle ist.

## Revendications

1. Trocart comportant :
une partie de canule (10) ayant une extrémité (11) coupée en biais à l'extrémité de la partie de canule, et
- un élément d'incision (A) qui est inséré de manière détachable dans la partie de canule et comporte une partie de bord de coupe (2) ayant une arrête de coupe formée à l'extrémité,
élément d'incision comprenant :
a) un corps principal,
b) une extrémité,
c) une partie amincie (5) formée dans une position prédéterminée du côté de l'extrémité de l'élément d'incision,
d) une partie de tige mince (6) entre l'extrémité de l'élément d'incision et la partie amincie,
trocart adapté à la chirurgie ophtalmique,
- la forme de la projection de l'arrête de coupe de l'élément d'incision dans un plan perpendiculaire à l'axe longitudinal étant une ligne droite ou une ligne courbe, et
**caractérisé en ce que**
- l'extrémité inclinée (11) est réalisée dans la forme amincie, et
- le plus grand des diamètres de l'extrémité de l'élément d'incision est plus grand que le diamètre de la partie d'axe mince et il est égal au diamètre du corps principal de l'élément d'incision.

2. Trocart selon la revendication 1,
dans lequel
a) l'extrémité de l'élément d'incision comporte entre 1 et 4 parties inclinées (4a - 4d) formées à l'extrémité,
b) l'arrête de coupe fonctionnant pour inciser un tissu ayant :
(i) une première arrête là où la première inclinaison (4a) et la quatrième inclinaison (4d) se coupent,
(ii) une seconde arrête là où la seconde inclinaison (4b) et la troisième inclinaison (4c) se coupent, et
c) un sommet ayant :
(i) une troisième arrête là où la première inclinaison (4a) et la seconde inclinaison (4b) se coupent, et
(ii) une quatrième arrête là où la troisième inclinaison (4c) et la quatrième inclinaison (4d) se coupent, et
le sommet n'a pas pour fonction d'inciser un tissu mais juste la fonction d'élargir l'incision formée par la partie d'arrête de coupe,
- les deux extrémités de l'arrête de coupe et les deux extrémités du troisième et quatrième bord étant plus grandes que le diamètre de la partie d'axe mince et égales au diamètre du corps principal de l'élément d'incision.

3. Trocart selon la revendication 1,
dans lequel
l'angle de l'extrémité inclinée de la partie en forme de canule est d'environ 60° par rapport au centre de la tige.

4. Trocart selon la revendication 1,
dans lequel
la projection de l'arrête de coupe dans le plan perpendiculaire à l'axe longitudinal est n'importe qu'elle ligne droite passant par le centre de l'élément d'incision, une ligne droite passant par une position décalée par rapport au centre et une ligne courbe passant par une position décalée par rapport au centre.

5. Trocart selon la revendication 1,
dans lequel
la longueur de la projection de l'arrête de coupe est de 1,5 fois ou moins, égale au diamètre intérieur de la canule.
